(19) Europäisches Patentamt
European Patent Office
Office européen des brevets

(11) EP 0 582 318 B1

(12) # FASCICULE DE BREVET EUROPEEN

(45) Date de publication et mention de la délivrance du brevet: **22.09.1999 Bulletin 1999/38**

(51) Int. Cl.$^6$: **A61K 31/19**

(21) Numéro de dépôt: **93115509.7**

(22) Date de dépôt: **06.10.1990**

(54) **Utilisation de l'acide carnosique pour ses propriétés anticarcinogènes et antivirales**

Verwendung von Carnosolsäure wegen seiner anticarcinogenen und antiviralen Eigenschaften

Use of carnosic acid for its anticarcinogenic and antiviral properties

(84) Etats contractants désignés:
**AT BE CH DE DK ES FR GB GR IT LI LU NL SE**

(43) Date de publication de la demande:
**09.02.1994 Bulletin 1994/06**

(62) Numéro(s) de document de la (des) demande(s) initiale(s) en application de l'article 76 CBE:
**90119218.7 / 0 480 077**

(73) Titulaire:
**SOCIETE DES PRODUITS NESTLE S.A.**
**1800 Vevey (CH)**

(72) Inventeurs:
- **Aeschbach, Robert**
  **CH-1800 Vevey (CH)**
- **Philippossian, Georges**
  **CH-1010 Lausanne (CH)**

(74) Mandataire: **Thomas, Alain et al**
**55, avenue Nestlé**
**1800 Vevey (CH)**

(56) Documents cités:
**EP-A- 0 269 545**

- **ZEITSCHRIFT FüR LEBENSMITTEL-UNTERS. UND -FORSCHUNG vol. 141, no. 1 , 1969 pages 10 - 16 C.H. BRIESKORN ET AL. 'Carnosolsäure, der wichtige antioxydativ wirksame Inhaltsstoff des Rosmarin- und Salbeiblattes'**
- **J. PHARM. SCI. vol. 69, no. 12 , 1980 pages 1455 - 1457 R. EL-RASHIDY ET AL. 'Comparative pharmacokinetics of butylated hydroxyanisole and butylated hydroxytoluene in rabbits'**
- **J INFECT. DIS. vol. 138, no. 1 , 1978 pages 91 - 94 K.S. KIM ET AL. 'Inactivation of Cytomegalovirus and Semliki Forest Virus by Butylated Hydroxytoluene'**
- **R. BERKOW ET AL. 'The Merck Manual' 1987 , MERCK SHARP & DOHME RESEARCH LAB. , RAHWAY, N.J. * page 180 - page 183 * * see especially page 182 ***
- **VESTN. OFTALMOL. vol. 104, no. 4 , 1988 pages 65 - 68 V.S. RYKUN ET AL. 'Clinical and experimental validation for the use of alpha-tocopherol in the treatment of ophthalmic herpes'**
- **MUTATION RESEARCH vol. 269, no. 2 , 1992 pages 193 - 200 M. MINNUNNI ET AL. 'Natural antioxidants as inhibitors of oxygen species induced mutagenicity'**

EP 0 582 318 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.7/3.6

## Description

L'invention concerne l'utilisation de l'acide carnosique pour ses propriétés anticarcinogènes et antivirales.

L'acide carnosique est un diterpène phénolique répondant à la formule brute C20H2804 et possédant la structure I

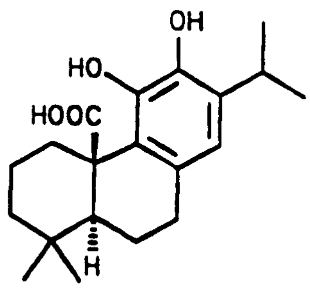

I

C'est un constituant propre aux espèces Salvia et Rosmarinus, où il est localisé principalement dans les feuilles. Il a été trouvé pour la première fois par Linde dans Salvia officinalis [Helv. Chim. Acta 47, 1234 (1962)] et par Wenkert et al. dans Rosmarinus officinalis [J. Org. Chem. 30, 2931 (1965)]. Par la suite, il a été identifié positivement dans diverses autres espèces de sauge, comme par exemple Salvia canariensis [Savona et Bruno, J. Nat. Prod. 46, 594 (1983)] ou Salvia willeana [de la Torre et al, Phytochemistry 29, 668 (1990). Il est également présent dans Salvia triloba et Salvia sclarea.

L'acide carnosique est un antioxydant puissant [Brieskorn et Dömling, Z. Lebensm. Unters. Forsch. 141, 10 (1969)] et, selon une série de travaux russes, où il porte le nom de salvine, un antibiotique contre Staphylococcus aureus [CA 86, 117603r; 90, 49011b; 97, 67513r, 69163a, 69164b; 104, 221930w; 111, 130594t] et contre certains microorganismes responsables de la carie des dents et de la mauvaise haleine [CA 97, 84835q]. L'art antérieur le cite au sujet de cette dernière propriété pour la préparation de dentifrices et d'eaux pour les soins de la bouche [JP 59'103'665, Lion Corp.].

Malgré cette abondance de citations, l'isolation à l'échelle préparative de l'acide carnosique à partir du romarin ou de la sauge n'a jamais été décrite, ni chez Linde ou Wenkert, où son existence est prouvée de façon indirecte, ni chez ceux qui l'ont identifiée par la suite dans diverses espèces de sauge.

En revanche, plusieurs autres diterpènes phénoliques de structure voisine de celle de l'acide carnosique ont été isolés de l'une ou/et l'autre des deux espèces Salvia et Rosmarinus. Parmi ceux-ci, il faut mentionner le carnosol Brieskorn et al, Chem. Ber. 95, 3034 (1962); J. Org. Chem. 29, 2293 (1964)], et plus récemment le rosmanol [Inatani et al., Agric. Biol. Chem. 46, 1661 (1982) ou le rosmaridiphénol [Houlihan et al., J. Am. Oil Chem. Soc. 61, 1036 (1984], ces deux derniers ayant été brevetés comme nouveaux principes antioxydants du romarin [USP 4'450'097, Nakatani et al., Lion Corp., Tokyo; USP 4'638'095, Chang et al., Research Corp., N.Y.].

Bien que tous ces composés soient dignes d'intérêt comme antioxydants, il n'en demeure pas moins que, comparés à l'acide carnosique, ils présentent certains aspects peu favorables: leur teneur dans le romarin ou la sauge est bien inférieure à celle de l'acide carnosique.

On connaît également des composés phénoliques ayant une activité antioxydante. L'article de Journal of Pharmaceutical Science, Vol 69, No. 12, Décembre 1980, pages 1455-1457, mentionne l'utilisation comme agent anti-cancéreux de l'hydroxyanisole butylé et de l'hydroxytoluène butylé. Le Journal of Infections Desease, Vol. 138, No. 1, Juillet 1978, pages 91-94, mentionne l'utilisation d'hydroxytoluène butylé pour des traitements viraux. Le brevet EP 0269545 mentionne l'utilisation de vitamine E pour le traitement du cancer. L'article de Vestn. Oftalmol. Vol. 104, No. 4, 1988, pages 65-68, mentionne l'utilisation de alpha-tocophérol pour le traitement de l'herpès.

Des feuilles séchées de romarin ou de sauge (espèce Salvia officinalis) contiennent entre 1,5 et 2,5% d'acide carnosique et seulement env. 0,3-0,4% de carnosol. Le indétectables. Du point de vue de l'économie d'un procédé d'obtention, l'acide carnosique présente donc un avantage indiscutable. Selon les indications données dans le brevet US 4'450'097 par exemple, on peut calculer que le rendement en rosmanol isolé à partir du romarin est de 0,01% seulement.

Ainsi qu'il a été montré par Wenkert et al., le carnosol est un artefact oxydatif de l'acide carnosique. Cette oxydation a lieu en présence d'oxygène, aussi bien après la récolte du romarin ou de la sauge dans les feuilles laissées

sécher à l'air (on peut d'ailleurs montrer que les feuilles fraîchement coupées de romarin ne contiennent pas de carnosol), que lorsque ces feuilles sont soumises à des opérations d'extraction aux solvants ou que les extraits eux-mêmes sont soumis aux opérations classiques de fractionnement, d'enrichissement et de purification. Il y a tout lieu de penser que le rosmanol, qui a été identifié dans une fraction de romarin soumise à un traitement alcalin, est lui aussi un produit ultérieur de l'oxydation de l'acide carnosique, ainsi que Wenkert et al. le suggéraient déjà, comme on pourrait raisonnablement aussi le supposer du rosmaridiphénol. L'acide carnosique est donc le seul diterpène phénolique présent à l'état natif dans le romarin et la sauge et à ce titre a seul droit à l'appellation de produit naturel.

Certaines voies d'obtention de l'acide carnosique par synthèse chimique ont aussi été proposées dans la littérature par W.L. Meyer et al. [Tetrahedron Letters 1966, 4261; 1968, 2963; J. Org. Chem. 41, 1005 (1976)]. Cependant, il s'agit de voies de synthèse longues et complexes, inapplicables pour des raisons économiques à un procédé industriel. De plus, ces synthèses conduisent à des mélanges racémiques de précurseurs de l'acide carnosique et non aux énantiomères purs. Il faut encore mentionner que ces travaux s'arrêtent à la préparation de précurseurs de l'acide carnosique et omettent de décrire la ou les étapes finales de préparation. Un autre mode d'obtention de l'acide carnosique a aussi été décrit dans la littérature par Brieskorn et Dömling [Arch. Pharm. 302, 641 (1969)] consistant en réduction catalytique de carnosol. Ici encore, une application à grande échelle de cette procédure ne pourrait être envisagée à cause de la non disponibilité du carnosol.

Un procédé d'obtention d'acide carnosique à partir de romarin ou de la sauge, objet de la demande principale, est caractérisé par le fait qu'on extrait l'épice avec un solvant non polaire ou un mélange de solvants à forte apolarité, on soumet l'extrait obtenu à un traitement d'adsorption sélective sur un support solide, on désorbe l'acide carnosique avec un solvant polaire ou un mélange de solvants à forte polarité et on évapore le solvant.

L'acide carnosique, comme toute molécule de type catéchol (ortho-diphénol), est un composé réactif, très sensible à l'oxydation, et donc très sensible aussi vis-à-vis de toutes les opérations usuellement pratiquées, pour l'isolation des substances naturelles (extraction, partition liquide-liquide, fractionnement chromatographique, etc.). En revanche, il a été trouvé que, sous sa forme purifiée et cristallisée, l'acide carnosique est stable et peut être manipulé sans précautions excessives, mais que cette obtention sous forme cristallisée, ne peut être réalisée qu'à partir d'une préparation de matériel végétal déjà enrichie en acide carnosique.

Le procédé selon la demande principale permet de préserver l'intégrité chimique de l'acide carnosique, car il ne comporte que deux étapes de traitement, ces étapes ménagent le produit de base et elles sont sélectives vis-à-vis de l'acide carnosique.

Le procédé d'obtention de l'acide carnosique exploite deux propriétés physico-chimiques de l'acide carnosique. D'une part, la molécule comporte des fonctions réputées polaires comme la fonction acide carboxylique et phénol. D'autre part, le reste de son squelette, essentiellement hydrocarboné, lui confère un caractère relativement apolaire en comparaison de tous les autres composés phénoliques, comme les flavonoïdes ou les acides hydroxybenzoïques ou hydroxycinnamiques, dont sont abondamment pourvus des végétaux comme la sauge ou le romarin. Ainsi, le procédé comporte seulement deux opérations:

1. Extraction du matériel végétal (sauge ou romarin) dans un solvant essentiellement apolaire, par quoi l'acide carnosique et les composés apolaires du matériel végétal passent dans l'extrait.

2. Adsorption sélective de l'acide carnosique contenu dans l'extrait sur un support solide choisi pour son affinité et sa sélectivité à l'endroit des fonctions polaires de l'acide carnosique, adsorption suivie de la désorption de l'acide carnosique du support à l'aide d'un solvant polaire.

Dans la première opération, des feuilles de romarin ou de sauge sont extraites par un solvant essentiellement apolaire, de manière à ce que l'extrait obtenu contienne, à côté de tous les autres composés apolaires ou très peu polaires des feuilles de ces plantes, comme les composants de l'huile essentielle, les lipides, les cires, des pigments chlorophylliens et certains triterpènes, l'acide carnosique comme pratiquement l'unique composé de type phénolique passant dans l'extrait. Le taux d'extraction de l'acide carnosique se situe entre 70 et 100% et sa teneur dans l'extrait est comprise entre 13 et 25%.

Dans la deuxième opération, l'extrait précédent est traité avec une matière solide adsorbante ayant de l'affinité pour les composés comportant des fonctions polaires ou présentant une sélectivité particulière à l'endroit des composés phénoliques, comme par exemple le gel de silice, l'oxyde d'aluminium, pour citer des matières absorbantes inorganiques, ou la polyamide, le polyvinylpyrolidone pour citer des exemples de matières absorbantes organiques. Durant ce traitement, l'acide carnosique est adsorbé avec une grande affinité ou sélectivité sur l'adsorbant, les autres constituants de l'extrait demeurant essentiellement dans la phase liquide. Celle-ci écartée, l'acide carnosique est désorbé de l'adsorbant par contact avec un solvant polaire, qui après évaporation donne un résidu contenant entre 65 et 95% d'acide carnosique dont, si nécessaire, la purification peut encore être parachevée par recristallisation.

Le rendement en acide carnosique du procédé d'obtention, calculé par rapport à la teneur de cet acide dans

la matière végétale de départ, est compris entre 60 et 90%.

La matière végétale de départ et la taille des particules végétales jouent un rôle dans le rendement d'extraction de l'acide carnosique. En principe, on préférera utiliser du romarin ou de la sauge finement moulus, une mouture fine donnant généralement de meilleurs rendements. Bien qu'il sera plus avantageux de partir de l'épice entière, les résidus obtenus après l'hydrodistillation de l'huile essentielle pourront le cas échéant aussi faire l'affaire, mais il faut savoir que, cette opération se soldant par des pertes importantes d'acide carnosique sous forme de produit d'oxydation, le procédé d'obtention de l'acide carnosique sera économiquement moins favorable. On pourra aussi utiliser comme matière première les résidus obtenus après l'extraction de l'huile essentielle avec un solvant en phase supercritique, comme le C02 supercritique par exemple. Dans ce cas, les résidus seront de meilleure qualité du point de vue de leur contenu en acide carnosique, car cette technique d'obtention de l'huile essentielle ménage mieux les composés précieux de la matière végétale.

D'une manière générale, l'acide carnosique s'extrait plus aisément de la sauge que du romarin. L'explication réside certainement dans le fait que les feuilles de sauge sont moins fibreuses que les feuilles de romarin. Le romarin est cependant une plante beaucoup plus répandue que la sauge, et constitue donc une matière de départ moins onéreuse et plus facilement disponible en grande quantité.

Quelle que soit le type d'extraction ou la matière végétale mise en oeuvre, il importera, pour les raisons décrites plus haut, que le solvant d'extraction soit aussi apolaire que possible. Il s'agira de solvants couramment utilisés comme solvants d'extraction, c'est-à-dire de points d'ébullition relativement bas, comme les hydrocarbures saturés, ramifiés ou non, par exemple hexane, pentane, heptane, 2-méthyl-butane, 2 méthyl-hexane et cyclohexane ou les mélanges d'hydrocarbures saturés (éthers de pétrole), ou les hydrocarbures aromatiques comme le toluène, ou encore de mélanges binaires de l'un des solvants précédents en fort excès avec un solvant chloré (par exemple chlorure de méthylène, chloroforme, chloroéthylènes) ou oxygéné comme un éther (p. ex. éther diéthylique), ou une cétone (p. ex. acétone), ou un ester (p. ex. acétate d'éthyle), ou un alcool (p. ex. éthanol, méthanol, etc.). On peut utiliser par exemple un mélange d'éthers de pétrole, de toluène ou d'hexane avec le dichlorométhane ou l'éthanol en proportions 99/1 à 90/10. Dans le cas de la sauge, par exemple, il sera possible d'extraire quantitativement l'acide carnosique avec un solvant de type hydrocarbure saturé. Avec toutes les autres combinaisons matière végétale/variante d'extraction, il faudra avoir recours à un solvant de polarité légèrement augmentée si l'on souhaite obtenir un bon taux d'extraction d'acide carnosique.

L'étape d'extraction de l'épice peut être réalisée soit par épuisement au solvant de la matière végétale, par exemple dans un extracteur de type Soxhlet (Variante I), soit par charge, c'est-à-dire par immersion de la matière végétale dans le solvant (Variante II), soit par percolation, soit par extraction en colonne pulsée, soit par toute autre technique connue d'extraction solide par solvant. Ce sont cependant les deux premières variantes qui seront exemplifiées ci-dessous. La variante I donne de meilleurs taux d'extraction d'acide carnosique que la variante II. Celle-ci est toutefois plus commode à mettre en oeuvre quand il faut extraire des quantités importantes de matière végétale.

L'extraction par épuisement au solvant se réalise dans un extracteur de type Soxhlet composé d'un dispositif de reflux (ballon + réfrigérant), qui contient le solvant d'extraction et entre les deux parties duquel est intercalé un extracteur à syphon muni d'une cartouche poreuse contenant la matière végétale à extraire. Les vapeurs du solvant chauffé à l'ébullition dans le ballon cheminent le long de l'extracteur dans un conduit aménagé à cet effet et se condensent en liquide une fois parvenues dans le réfrigérant. Le solvant condensé retombe dans la cartouche d'extraction qu'il remplit. Une fois le niveau de liquide dans l'extracteur parvenu à la hauteur du conduit de syphonnage, l'extracteur se vide de son contenu en liquide, qui retourne au ballon, emportant avec lui une certaine quantité de matière végétale dissoute. L'ensemble du processus peut être défini comme un cycle d'extraction.

Selon ce mode d'extraction, les solvants préférés sont pour la sauge les hydrocarbures légers acycliques, par ex. l'éther de pétrole, de préférence à intervalle d'ébullition 40-60°C et pour le romarin les mêmes solvants ou une combinaison binaire de l'un de ceux-ci avec un solvant chloré, par exemple le dichlorométhane, en proportions volumiques de 99/l jusqu'à 9/l. Le nombre de cycles d'extraction appliqués à la matière à extraire sont de l'ordre de 5-20.

Si on extrait par charges la matière végétale et le solvant d'extraction sont mis en contact dans un réacteur conventionnel et le mélange est brassé durant toute l'opération. Les proportions solvant/matière végétale sont de préférence dans un rapport volume/poids de 5/l dans le cas du romarin, et de 10/l dans le cas de la sauge (la quantité plus importante de solvant dans le cas de sauge est commandée par la masse volumique beaucoup plus faible de celle-ci comparée à celle du romarin). Généralement, on procède à 2 ou 3 extractions successives de la matière végétale, après avoir entre chaque opération séparé la phase liquide et la phase solide par filtration ou centrifugation. La durée de chaque extraction est de l'ordre de 1/2 à 2 heures, généralement de 1 heure. Les solvants préférés dans ce mode d'extraction sont pour les deux matières végétales sauge et romarin les hydrocarbures aromatiques, de préférence le toluène, ou une combinaison binaire d'un hydrocarbure léger acyclique, par ex. l'éther de pétrole ou l'hexane, avec un solvant oxygéné, de préférence l'éthanol ou le méthanol, en proportions volumiques de 99/l jusqu'à 9/l.

La première étape d'extraction est effectuée à une température comprise entre 20 et 50°C.

Dans la seconde étape, l'extrait végétal obtenu lors de l'étape précédente est traité avec une matière adsorbante solide. L'acide carnosique de l'extrait est sélectivement adsorbé sur la matière solide, puis, la phase liquide écartée, est récupéré à l'état concentré par désorption avec un solvant polaire pur, tel que l'acétone, le méthanol, l'éthanol ou l'acétate d'éthyle, ou un mélange de l'un de ceux-ci en proportion importante avec un solvant apolaire ou peu polaire.

En principe, l'extrait liquide pourra être traité par n'importe quelle matière solide adsorbante ayant de l'affinité ou de la sélectivité pour ce type de composés. Une liste non exhaustive des matières adsorbantes pouvant être utilisées à cet effet a déjà été donnée ci-dessus. Ces matières sont essentiellement celles que l'on utilise couramment dans les techniques de séparation par chromatographie liquide.

Parmi les matières adsorbantes économiquement intéressantes, la polyamide ou tout polymère similaire, comme la polyvinylpyrrolidone, sont des matières de choix pour l'adsorbtion de l'acide carnosique. Ces matières en effet montrent une affinité tout à fait remarquable à l'endroit des composés phénoliques (cf. p. ex. "The Flavonoids", Harborne et al., eds., Chapman and Hall, 1975, Chap. 1, p. 11). De plus, ce sont des supports inertes chimiquement qui ne risquent pas d'altérer sensiblement les composés avec lesquels elles sont mises en contact.

Pratiquement, l'extrait végétal liquide de la première étape du procédé peut être mis en contact tel quel avec la matière adsorbante. Le cas échéant, l'extrait aura été auparavant filtré pour le débarrasser de petites quantités de matières solides précipitées qui auraient pu éventuellement se former durant l'extraction ou postérieurement à celle-ci. Pratiquement cependant, on aura avantage à concentrer l'extrait liquide avant sa mise en contact avec l'agent adsorbant de manière à favoriser le passage de l'acide carnosique de l'état dissous à l'état adsorbé. Dans de nombreux cas, quand l'opération de concentration se traduit par la formation d'un précipité solide, on préférera plutôt chasser complètement le solvant de l'extrait et reprendre le résidu dans un deuxième solvant choisi pour son aptitude à dissoudre facilement et complètement l'acide carnosique de l'extrait. Dans la pratique, on a trouvé que les solvants de type hydrocarbure aromatique ou chlorés faisaient bien l'affaire pour cette opération, le toluène et le dichlorométhane étant les solvants préférés.

La mise en contact de l'extrait liquide avec la matière adsorbante peut se réaliser par immersion ou par passage de l'extrait à travers une colonne remplie de la matière adsorbante. Cette seconde technique est plus efficace et on opère alors de la manière suivante: l'extrait liquide est chargé au sommet d'une colonne remplie avec de la matière adsorbante conditionnée avec le même solvant que celui de l'extrait. Une fois l'extrait en contact avec la matière adsorbante, on lave la colonne avec du solvant frais jusqu'à ce que toutes les matières de l'extrait aient été éliminées sauf l'acide carnosique qui reste adsorbé sur le support. L'acide carnosique est alors désorbé de la matière adsorbante en faisant passer à travers la colonnne un solvant moyennement polaire à polaire par exemple un mélange du dichlorométhane ou de toluène avec de l'éthanol ou du méthanol. On chasse le solvant de l'éluat et le résidu peut être encore purifié ultérieurement par recristallisation pour atteindre le degré de pureté souhaité en acide carnosique.

L'invention concerne l'utilisation de l'acide carnosique pour la fabrication d'une composition ou d'une diète destinée à prévenir ou traiter le cancer.

Certains composés chimiques possèdent des propriétés qui, de manière directe ou indirecte, réduisent ou suppriment l'activité mutagène induite par d'autres produits chimiques. De fait, il a été montré que les radicaux libres sont capables d'induire un grand nombre de lésions différentes sur l'ADN et qu'ils sont aussi impliqués dans le processus du cancer, du vieillissement et des maladies cardiovasculaires. L'acide carnosique a une action inhibitrice de dégradation de l'ADN causée par les radicaux libres, ce qui permet d'envisager son utilisation pour la prévention et le traitement de maladies cancéreuses ou cardiovasculaires.

Les compositions diététiques ou pharmaceutiques peuvent se présenter sous différentes formes adaptées au mode d'administration, par exemple par voie orale, entérale ou parentérale. On peut par exemple préparer des capsules, gélules ou sirops. Dans le cas d'une administration entérale ou parentérale, les compositions se présentent sous forme de solutions ou émulsions stabilisées physiquement et chimiquement.

Des doses physiologiques peuvent être administrées dans la prévention ou éventuellement le traitement de certaines formes du cancer et des maladies cardiovasculaires.

L'acide carnosique peut en outre être utilisé pour la fabrication d'une composition destinée au traitement de l'herpès, qui est une affection virale. Cette composition peut se présenter sous différentes formes adaptées au mode d'administration, par exemple par voie orale ou par application topique. On peut par exemple préparer des capsules, gélules ou pommades. Des doses physiologiques sont administrées pour le traitement de cette affection.

La suite de la description est faite en référence aux exemples.

<u>Exemples 1 à 21</u>

Le Tableau 1 ci-dessous illustre les résultats obtenus à partir d'une série d'essais d'extraction de romarin et de sauge selon les variantes d'extraction I et II en utilisant les solvants décrits ci-dessus. On voit que plus la polarité du solvant augmente (colonne 5), meilleur est le taux d'extraction de l'acide carnosique (colonne 8), mais moins aussi sa concentration dans l'extrait est importante (colonne 7) et donc plus le ballast dans l'extrait est considérable, ce ballast

étant notamment formé d'autres composés phénoliques que l'acide carnosique et donc susceptibles d'interférer négativement avec celui-ci dans la seconde étape du procédé.

| 1 | 2 | 3 | 4 | 5 | 6 | 7 | 8 | 9 |
|---|---|---|---|---|---|---|---|---|
| Exemple | Mat. prem. | Teneur AC (%) | Variante extraction | Solvant extraction | Rdmt extraction extrait (%) | Taux AC dans (%) | Taux extraction AC (%) | IQ extr. (max-100) |
| 1 | Romarin | 1,85 | I | P | 9,1 | 16 | 79 | 68 |
| 2 | Romarin | 1,85 | I | P/D 9/1 | 10,1 | 16 | 87 | 76 |
| 3 | Romarin | 1,85 | I | P/D 9/1 | 11,2 | 15 | 91 | 74 |
| 4 | Sauge | 2,50 | I | P | 10,0 | 25 | 100 | 100 |
| 5 | Romarin | 1,80 | II | P | 5,8 | 13 | 42 | 30 |
| 6 | Romarin | 1,80 | II | T | 9,6 | 14 | 75 | 58 |
| 7 | Romarin | 1,80 | II | P/E 98/2 | 8,2 | 16 | 73 | 65 |
| 8 | Romarin | 1,80 | II | P/E 99/1 | 7,2 | 15 | 60 | 50 |
| 9 | Romarin | 1,80 | II | P/E 98/2 | 8,2 | 15 | 68 | 57 |
| 10 | Romarin | 1,80 | II | P/E 95/5 | 11,4 | 13 | 82 | 59 |
| 11 | Romarin | 1,80 | II | P/A 98/2 | 6,4 | 13 | 46 | 33 |
| 12 | Romarin | 1,80 | II | P/A 95/5 | 7,6 | 14 | 59 | 46 |
| 13 | Romarin | 1,80 | II | P/A 9/1 | 10,0 | 12 | 67 | 44 |
| 14 | Sauge | 1,80 | II | P | 6,0 | 16 | 53 | 47 |
| 15 | Sauge | 1,80 | II | P/E 99/1 | 9,0 | 15 | 75 | 63 |
| 16 | Sauge | 1,80 | II | P/E 98/2 | 9,0 | 15 | 75 | 63 |
| 17 | Sauge | 1,80 | II | P/E 95/5 | 13,4 | 11 | 82 | 50 |
| 18 | Sauge | 1,80 | II | T | 10,2 | 16 | 91 | 82 |
| 19 | Sauge | 1,80 | II | P/A 98/2 | 6,8 | 14 | 53 | 41 |
| 20 | Sauge | 1,80 | II | P/A 95/5 | 8,4 | 16 | 75 | 66 |
| 21 | Sauge | 1,80 | II | P/A 9/1 | 11,6 | 13 | 84 | 61 |

Explications des abréviations:
AC: Acide carnosique
IQ: Indice de qualité
Solvants:
P = Ether de pétrole
E = Ethanol
A = Acétone
D = Dichlorométhane
T = Toluène

On peut définir à partir des données mesurées du tableau un indice de qualité (IQ) de l'extrait, indice déterminé à partir de critères d'efficacité et de sélectivité du solvant d'extraction. L'efficacité (E) du solvant est mesurée par le taux d'extraction de l'acide carnosique (colonne 8). Plus le solvant est efficace, meilleur sera le rendement en acide carnosique récupéré en fin de procédé. La sélectivité (S) du solvant d'extraction est mesurée par la teneur de l'acide carnosique dans l'extrait (colonne 7), plus le solvant est sélectif, meilleure sera la pureté de l'acide carnosique isolé en fin de procédé. L'indice de qualité (IQ) peut alors être défini comme le produit des facteurs E et S, produit pondéré par le taux d'acide carnosique (T) dans la matière végétale de départ (colonne 3).

$$IQ = (E \times S) / T$$

Pour faciliter la comparaison entre les différents exemples du Tableau 1, l'indice de qualité (colonne 9) a été relativisé à une échelle de 100, en considérant arbitrairement comme optimales les données de l'exemple 4.

L'examen des données du tableau permet de faire les observations suivantes:

1) L'indice de qualité est l'expression d'un compromis entre sélectivité et efficacité. Le solvant idéal, serait celui qui extrairait l'intégralité de la matière active recherchée et seulement celle-là. Le compromis à réaliser consiste à trouver un équilibre entre le rendement du procédé d'obtention de la matière active et l'économie de ce procédé.

2) A mises en oeuvre comparables de variante d'extraction et de solvant, l'acide carnosique s'extrait plus aisément de la sauge que du romarin.

3) A mise en oeuvre comparable de solvant et quelle que soit la matière première végétale, la variante d'extraction I est plus efficace que la variante II.

4) La mise en oeuvre de mélanges binaires de solvants d'extraction à proportion croissante du plus polaire d'entre eux permet de définir une concentration optimale de ce dernier qui se situe autour de 5% dans le cas des exemples donnés dans le tableau (exemples 7-9, 10-12, 15-17, 18-19).

La suite de la description donne des exemples concrets d'extraction d'acide carnosique selon les exemples 4, 1, 2, 7 et 18 du tableau 1 précédent.

<u>Sauge, extraction variante I, selon exemple 4</u>

Dans un extracteur de type Soxhlet muni d'une cartouche de cellulose, on place 297 g de sauge officinale moulue contenant 2,5% d'acide carnosique. On extrait durant 48 h à l'abri de l'air (atmosphère d'azote) avec de l'éther de pétrole (2,5 L; PE 40-60°C). L'extraction terminée, on chasse le solvant à l'évaporateur rotatif et on recueille 30 g d'extrait huileux coloré (Rendement 10%), contenant 7,4 g (Rendement 100%) d'acide carnosique.

On dissout l'extrait dans du dichlorométhane (150 ml) et, après avoir filtré cette solution pour éliminer une faible proportion de matière insoluble, on la verse sur une colonne remplie de polyamide et préparée à partir d'une suspension de 150 g de ce matériel dans 1 l de dichlorométhane. On élue avec ce même solvant pour éliminer les matières de l'extrait non retenus sur la polyamide et correspondant à une fraction fortement colorée (Fraction 1, 700 ml, 18 g de résidu sans solvant. On poursuit l'élution avec un mélange 8/2 (v/v) de dichlorométhane/méthanol. La zone de transition entre les deux solvants se manifeste sur la colonne par la présence d'une zone jaune en forme d'anneau, qui correspond à l'acide carnosique. On recueille une fraction intermédiaire (Fraction 2, 700 ml, 2 g de résidu sans solvant), puis la zone de l'anneau (Fraction 3, 100 ml, 6,1 g de résidu après avoir chassé le solvant).

Le résidu semi-cristallin de la fraction 3 après trituration dans de l'éther de pétrole donne 6,0 g d'un solide jaune-clair (F 170-195°C) contenant 95% d'acide carnosique. Rendement: 82%.

<u>Romarin, extraction variante I selon exemple 1</u>

On place dans le récipient d'extraction d'un extracteur de Soxhlet un bas de tissu mince contenant 383 g de romarin moulu dont la teneur en acide carnosique est de 1,85% en poids. La hauteur de la masse végétale dans l'extracteur mesure 30 cm. On place l'extracteur sous atmosphère inerte et on extrait avec de l'éther de pétrole (2,5 l; PE 40-60°C) par 4 cycles en tout de remplissage et syphonnage d'une durée de 75 min chacun. On chasse le solvant à l'évaporateur rotatif et on recueille 35 g d'extrait huileux foncé (Rendement 9,1%), qui contient 5,6 g (Rendement 79%) d'acide carnosique.

On dissout l'extrait dans 240 ml de dichlorométhane et on verse la solution sur une colonne de polyamide. On élue comme précédemment en collectant 3 fractions: Fr. 1: 700 ml; Fr. 2: 550 ml; Fr. 3: 500 ml.

On chasse le solvant de la dernière fraction et on obtient 7,3 g d'une masse solide jaune foncé, qui contient 5,6 g (77%) d'acide carnosique, soit la totalité de la fraction extraite de cet acide. Rendement: 79%.

<u>Romarin, extraction variante I selon exemple 2</u>

On opère comme dans l'exemple précédent, sauf qu'on extrait avec un mélange éther de pétrole/dichlorométhane 9/1 (v/v). L'extrait pèse 38 g (Rendement 10,1%) et contient 6,2 g (Rendement 87%) d'acide carnosique. Une partie de l'extrait (4 g) n'est pas soluble dans le dichlorométhane et il faut prendre soin de filtrer cette matière solide avant de passer l'extrait sur la colonne de polyamide. Le résidu de la fraction 3 de l'élution pèse 7,7 g et contient 5,3 g (59%) d'acide carnosique. Rendement: 74%.

Romarin, extraction variante II selon exemple 7

Dans un réacteur de 20 l, on introduit 2,5 kg de romarin moulu contenant 1,8% d'acide carnosique et 12,5 l d'un mélange 98/2 (v/v) d'hexane/éthanol. On brasse le tout pendant 1 heure à température ambiante et sous azote. On sépare la phase liquide de la phase solide par une filtration sous vide (Büchner). On met de côté la solution de l'extrait et on soumet la masse végétale à une deuxième extraction semblable à la première. Après filtration, on réunit les deux solutions d'extrait et on ôte le solvant à l'évaporateur rotatif. On obtient 206 g (Rendement 8.2%) d'extrait huileux foncé qui contient 33 g (Rendement 73%) d'acide carnosique.

On dissout l'extait dans 1,6 l de dichlorométhane et on verse la solution sur une colonne de polyamide. On élue comme sous l'exemple 1 en collectant 3 fractions: Fr. 1: 3,36 l; Fr. 2: 3,23 l; Fr. 3: 1,65 l. Cette dernière fraction donne après évaporation du solvant un résidu semi-huileux (41,5 g) qu'on triture dans de l'éther de pétrole jusqu'à consistance solide. Le produit jaune ainsi obtenu pèse 37 g et contient 73% d'acide carnosique. Rendement: 27 g (60%).

Sauge, extraction variante II selon exemple 18

On brasse pendant 1 heure à température ambiante et sous azote un mélange de 50 g de sauge moulue (Taux d'acide carnosique 1.8%) et de 600 ml de toluène. Après séparation des deux phases solide et liquide par filtration, on soumet la masse végétale à une deuxième opération d'extraction. On réunit les deux solutions d'extrait et on chasse le solvant à l'évaporateur rotatif. On obtient 5,1 g (10,2%) d'extrait huileux contenant 0,82 g (Rendement 91%) d'acide carnosique.

On redissout l'extrait dans 50 ml de toluène et on filtre la solution obtenue pour éliminer quelques matières non solubles. Le filtrat est versé sur une colonne de polyamide (30 g; 30 x 2 cm) conditionnée dans du toluène. On élue comme dans l'exemple 1 avec du toluène, puis avec un mélange toluène/éthanol 8/2, et on recueille 3 fractions: Fr. 1: 200 ml (3,3 g de matière végétale); Fr. 2: 160 ml; 0,2 g; Fr. 3: 50 ml; 1,1 g. Cette dernière fraction contient 0,78 g (71%) d'acide carnosique. Rendement: 87%.

Purification de l'acide carnosique

30 g du produit jaune obtenu selon l'exemple 7 et contenant 73% d'acide carnosique sont recristallisés deux fois dans du cyclohexane en présence de charbon actif. On obtient 16,4 g d'acide carnosique sous la forme de cristaux incolores avec une pureté supérieure à 95% Point de fusion 193-199°C). A la place du cyclohexane, le produit peut aussi être recristallisé dans le benzène ou le toluène.

Les propriétés physiologiques anticarcinogènes et antivirales de l'acide carnosique sont illustrés dans les essais suivants:

Activité anticarcinogène de l'acide carnosique

L'activité antimutagène de l'acide carnosique a été évaluée dans un test d'Ames dans lequel on utilise la souche de Salmonella typhimurium TA 102, qui est connue pour répondre facilement aux espèces oxygénées actives. Cette souche est mise en présence de peroxyde de tertiobutyle (tBOOH) connu pour générer des radicaux peroxylés et dont l'action biologique est considérée comme particulièrement intéressante puisqu'il génère les radicaux oxygène à l'intérieur des cellules. Le tBOOH produit une certain nombre d'altérations ponctuelles
sur l'ADN des bactéries et on mesure l'inhibition de ces altérations produite par l'antioxydant quand on l'incorpore au milieu de culture. Les antioxydants suivants ont été testés dans une gamme de doses actives: acide carnosique, carnosol et acide ascorbique. Ce dernier, dont l'activité anticarcinogène est bien connue, a servi de contrôle positif. Afin de dissocier l'effet antimutagène "vrai" d'un effet bactéricide, un test de préincubation est utilisé et les résultats sont exprimés sous la forme du rapport: Nombre de colonies révertantes induit/Nombre de colonies survivantes [Aeschbacher et al., Food Safety, 8, 167-177 (1987)].

Description du test: Le milieu d'incubation est préparé en mélangeant 1 ml de suspension bactérienne ($5 \times 10^9$ bact/ml) de Salmonella typhimurium TA 102, préparée selon Maron et Ames [Mutation Research, 113, 175-215 (1983)], 50 µl de tampon salin, 0,95 ml de KCl 0,15M et 2,8 ml de milieu de Davis-Mingioli supplémenté avec 24 µg d'histidine et 10 µg de biotine par ml. On ajoute ensuite 0,5 ml de solution de tBOOH (concentration finale 2,5 mM) et 0,5 ml de solution d'antioxydant dans l'eau pour l'acide ascorbique et dans des triglycérides à chaîne moyenne pour les antioxydants non hydrosolubles acide carnosique et carnosol. Le milieu est incubé à 37°C pendant 1 heure, puis après addition de 9 ml de bouillon nutritif à 0,8%, pendant encore 3 heures à 37°C pour fixer la mutation. Le milieu est ensuite centrifugé et les bactéries lavées resuspendues dans 3,5 ml de tampon salin.

Le compte des colonies de révertants et de survivants s'effectue sur des plaques d'agar nutritif en absence, respectivement en présence d'histidine, et incubées pendant 3 jours à 37°C, sur lesquelles on a déposé 0,1 ml de la

suspension bactérienne précédente. On opère le compte automatiquement sur un compteur Fisher count-All 800.

Le Tableau 2 exprime les résultats obtenus sous la forme des concentrations estimées d'antioxydants testés capables de réduire de moitié l'effet mutagène induit par le tBOOH à la concentration de 2.5 mM en l'absence d'antioxydants (concentration inhibitrice 50 = CI 50).

Tableau 2

| Entrée | Composé | CI50(mg) | CI50(mM) | Activité relative comparaison des CI50(mM) |
|---|---|---|---|---|
| 1 | Ac. carnosique | 0,3 | 0,15 | 100 |
| 2 | Carnosol | 17,8 | 9,3 | 2 |
| 3 | Ac. ascorbique | 2,8 | 2,7 | 6 |

Il ressort clairement des données du tableau que, alors que l'activité anticarcinogène du carnosol est un peu plus faible, mais d'ordre de grandeur comparable à celle de l'acide ascorbique (témoin positif), l'activité de l'acide carnosique est supérieure par un facteur d'environ 15 à celle l'acide ascorbique et de 50 à celle du carnosol.

Activité antivirale de l'acide carnosique

L'activité antivirale de l'acide carnosique a été testée in vitro à différentes concentrations (5; 2,5; 1,25; 0,62 μg/ml) contre des lots infectieux d'Herpes simple de type 1 (HSV1) et type 2 (HSV2), et de Poliovirus de type 3 (Polio 3), cultivés sur la lignée cellulaire VERO. Après 2 heures d'incubation, les titres obtenus sont comparés à celui des témoins sans inhibiteur (Test 1). Des aliquots de différents surnageants de tests sont ensuite inoculés sur des cellules neuves. Après 4 jours d'incubation, le comptage des particules virales permettra cette fois d'apprécier la production virale en présence de l'inhibiteur (Test 2). Le Tableau 3 présente les résultats de ces deux tests.

Tableau 3

| Concentration Ac. carnosique (μg/ml) | Nombre de particules virales/0,025 ml de surnageant | | | | | |
|---|---|---|---|---|---|---|
| | Test 1 | | | Test 2 | | |
| | HSV1 | HSV2 | POLIO 3 | HSV1 | HSV2 | POLIO 3 |
| 5 | 10 | 10 | $10^{12}$ | $10^{2}$ | <10 | $2 \times 10^{23}$ |
| 2,5 | 10 | 10 | $10^{12}$ | $10^{5}$ | $2 \times 10^{2}$ | $2 \times 10^{23}$ |
| 1,25 | $10^{3}$ | 10 | $10^{12}$ | $2.5 \times 10^{5}$ | $10^{5}$ | $10^{23}$ |
| 0,62 | $10^{4}$ | 10 | $10^{12}$ | $6 \times 10^{6}$ | $8 \times 10^{5}$ | $10^{22}$ |
| 0 (témoin) | $10^{5}$ | $10^{4}$ | $10^{12}$ | $2 \times 10^{8}$ | $10^{7}$ | $10^{23}$ |

Les résultats du test 1 montrent que la souche de virus Polio 3 n'est pas inhibée par l'acide carnosique. Par contre l'action anti-HSV1 et anti-HSV2 est tout à fait significative puisque des réductions de titre allant jusqu'à 3 à 4 facteurs de dix sont observées. Il y a donc une spécificité d'action de l'acide carnosique. Les résultats du test 1 sont confirmés par ceux du test 2: On constate que la production de Polio 3 peut être considérée comme équivalente quelle que soit la concentration d'acide carnosique. Par contre, la production de HSV1 et HSV2 est très affectée. Ceci confirme la spécificité d'action du produit, et aussi l'absence de toxicité cellulaire puisqu'à la plus forte concentration (5 μg/ml) la production de Polio 3 n'est pas modifiée.

## Revendications

1. Utilisation de l'acide carnosique pour la fabrication d'une composition ou d'une diète alimentaire destinée à prévenir ou traiter le cancer.

2. Utilisation de l'acide carnosique pour la fabrication d'une composition destinée au traitement de l'herpès.

## Claims

1. Use of carnosic acid for producing a composition or a dietary food designed to prevent or treat cancer.

2. Use of carnosic acid for producing a composition designed to treat herpes.

## Patentansprüche

1. Verwendung von Carnosinsäure zur Herstellung einer Zusammensetzung oder eines Diätnahrungsmittels, die für die Vorbeugung oder Behandlung von Krebs bestimmt sind.

2. Verwendung von Carnosinsäure für die Herstellung einer Zusammensetzung, die für die Behandlung von Herpes bestimmt ist.